Europäisches Patentamt

European Patent Office

Office européen des brevets

(11) Publication number: **0 120 111**
**A1**

## EUROPEAN PATENT APPLICATION

(21) Application number: **83103126.5**

(22) Date of filing: **29.03.83**

(51) Int. Cl.³: **C 12 M 1/04**

(43) Date of publication of application:
**03.10.84 Bulletin 84/40**

(84) Designated Contracting States:
**AT BE CH DE FR LI**

(71) Applicant: **MATE INC.**
**22-6, Tsukuda, 2-chome**
**Chuo-ku Tokyo(JP)**

(72) Inventor: **Kasugai, Makoto**
**B-807 Casa Aioi 22-6 Tsukuda 2-chome**
**Chuo-ku Tokyo(JP)**

(74) Representative: **Bühling, Gerhard, Dipl.-Chem. et al,**
**Patentanwaltsbüro Tiedtke-Bühling-Kinne**
**Grupe-Pellmann-Grams-Struif Bavariaring 4**
**D-8000 München 2(DE)**

(54) Process for culturing anaerobic bacteria and agents for preparing culture atmosphere.

(57) A process for culturing anaerobic bacteria in an atmosphere containing 10% carbon dioxide and no oxygen. An agent for producing such atmosphere comprises carbon dioxide-generating deoxidizer adapted to remove oxygen twice the volume of generated carbon dioxide.

EP 0 120 111 A1

SPECIFICATION


TITLE OF THE INVENTION

PROCESS FOR CULTURING ANAEROBIC BACTERIA
AND AGENTS FOR PREPARING CULTURE ATMOSPHERE


FIELD OF THE INVENTION

This invention relates to a process for culturing anaerobic, bacteria, particularly for preparing an anaerobic atmosphere of 10 % by volume of carbon dioxide and agents therefor.


BACKGROUND

Anaerobic bacteria in the present invention encompass all kinds of bacteria which suffer any adverse reaction towards oxygen.

Different manners have been disclosed for detecting the anaerobic bacteria by cultivating the anaerobic bacteria and such cultivation has been known to be useful as means for detecting different kinds of bacteria. A simple manner for preparing an adequate atmosphere for such cultivation is required especially for the clinical purpose. Various deoxidizers and carbon dioxide-generators have been developed, and various manners for preparing a culture atmosphere having a carbon dioxide content of 3 - 5 % by volume have been put to practical use. However, the conventional manners still have various drawbacks. Namely, some of them are that the number of the species of bacteria detectable by one cultivation test is limited, and that the detecting test procedure is complicated.

It is too troublesome particularly in the clinical use to weigh out a specified amount of deoxidizers and carbon dioxide-generators according to the volume of every culture vessels since a great number of test samples must be cultured, in the clinics depending upon daily needs. Thus there is much to be desired in the prior art.

## OBJECTS OF THE INVENTION

It is a main object of the present invention to overcome the named drawbacks in the prior art.

It is an object of the present invention to provide a simpler process and agents for producing an anaerobic atmosphere enabling to detect as many as possible typical species of bacteria by one cultivation test.

It is another object of the present invention to provide a process and agents for preparing an anaerobic atmosphere containing 10 % by volume of carbon dioxide simply by enclosing small packages of an atmosphere-adjusting agent in culture vessels.

## SUMMARY OF THE INVENTION

According to the present invention, an atmosphere containing not exceeding 0.1 % by volume of oxygen and 10 ± 2 % by volume of carbon dioxide is provided for culturing anaerobic bacteria. The present invention provides a process for preparing an anaerobic atmosphere by enclosing an agent enclosed in a gas-permeable inner bag, the inner bag being put in a sealed culture vessel, the agent being capable of deoxidizing, as well as generating carbon dioxide so adjusted as to produce such a relation that the volume of the removed oxygen is twice that of the generated carbon dioxide.

## BRIEF DESCRIPTION OF DRAWING

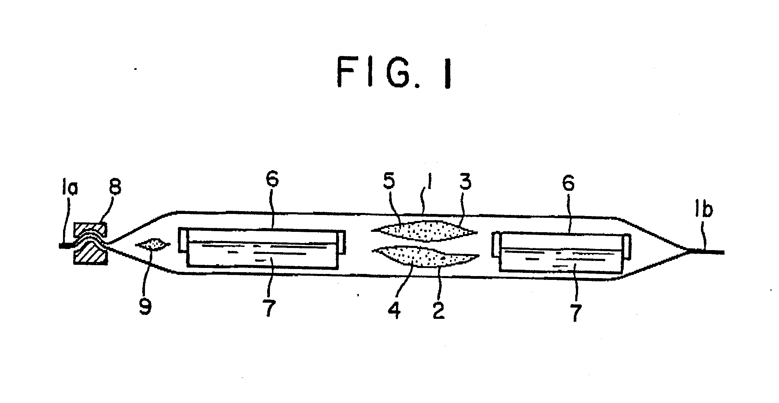

The drawing of a single Figure is a longitudinal sectional view of an embodiment of an anaerobic cultivation device of the invention.

The drawing is presented to demonstrate a preferred embodiment for better illustration of the invention and not for limitation thereof. In the following preferred embodiments will be described in detail, which however should not be construed to limit the invention thereto. Any modifications and changes as is apparent in the art may be made within the gist of the invention as claimed.

## DETAILED DESCRIPTION OF THE PREFERRED EMBODIMENTS

The present invention is based on the fact that most of typical anaerobic bacteria are well cultured in an atmosphere not only free of oxygen but containing about 10 % by volume of carbon dioxide.

It has been known in the art to use carbon dioxide-generators together with deoxidizers to regulate the carbon dioxide content to a level of 5 % by volume. According to this manner, Fusobacterium Nucleatum among eight typical species of the anaerobic bacteria has heretofor been impossible to be defected. However, when carbon dioxide is 10 % by volume, Fusobacterium Nucleatum can be also cultured and detected. Thus another caltivation test can be omitted for detecting all typical kinds of these bacteria. Further tests have proved that the process of the present invention realizes the detection of 30 typical kinds of bacteria including said 8 bacteria by culturing them. As a culture medium, fixed culture medium is suitable, however, other mediums are also applicable. Typical anaerobic bacteria which can be detected by the invention are as follows: Bacteroids, Fusobacterium, Clostridium, Propionibacterium, Lactobacillus, Peptococcus, Peptostreptococcus.and Veillonella. Six of them except Clostridium and Lactobacillus are known to be particularly clinically important from the pathogenic view point.

It has been confirmed that the inventive process is applicable to 30 kinds of anaerobic bacteria including above 8 kinds, and further study in progress may reveal further application.

The cultivation proceeds in an atmosphere containing not exceeding 0.1 % by volume (% refers to % by volume hereinafter) of oxygen and 10 ± 2 % of carbon dioxide during 24 - 48 hours at shortest after inoculation and preferably 6 - 48 hours. In this process deoxidizing and carbon dioxide-generating agent according to the present invention provides an ideal atmosphere (of less than 0.1 % oxygen with approximate 10 % carbon dioxide) within about 3 hours. Other agents reacting more slowly may be used in choice.

One type of the typical agents according to the invention has following composition:

(a)   ascorbic acid and/or its salts

(b)   carbonate(s) and/or hydrogen carbonate(s) of alkali, and

(c)   accelerator(s)

Ascorbic acid encompasses both of L-ascorbic acid and D-isoascorbic acid. The carbonates and hydrogen carbonates of alkali metal such as Na, Li, K and alkaline earth metal such as Mg, Ca etc. are suitable as the second component. Hydroxides of alkali metals and alkaline earth metals may be optionally added to the composition.

Some deliquescent salts such as calcium chloride may be also contained in the composition. As the accelerators, a mixture of ferrous salts and active carbon is most preferred, the amount of the accelerators being chosen according to the requisite reaction speed. Typical ferrous salts encompass ferrous sulfate, ferrous chloride, ferrous hydroxide, ferrous carbonate, and ferrous oxide, preferably hydrated ferrous sulfate. Powdered active carbon is conventionally produced by activating carbon with steam, $CO_2$, air, chloride or vacuum heating or according to other known manners.

Some examples of the basic composition of each agent are shown as follows:

| (A) | parts by weight | |
|---|---|---|
| | (A-1) | (A-2) |
| L-ascorbic acid | 14 | 14 |
| active carbon | 14 | 12 |
| $FeSO_4 \cdot 7H_2O$ | 30 - 50 | 58 |
| $CaCl_2 \cdot 2H_2O$ | 0 | 6 |
| $Ca(OH)_2$ | 0 | 3 |
| $NaHCO_3$ | 7 | 6 |
| $Na_2CO_3 \cdot 10H_2O$ | 7 | 6 |

Another example using powdery iron as a deoxidizer has following composition:

| (B) | parts by weight |
|---|---|
| powdery iron | 10 |
| $FeSO_4 \cdot 7H_2O$ | 56 |
| $Ca(OH)_2$ | 10 |
| $Na_2SO_3 \cdot 7H_2O$ | 7 |
| $NaHCO_3$ | 10 |
| $CaCl_2 \cdot 2H_2O$ | 7 |
| active carbon | 1.5 |

The compositions hereinabove mentioned remove or absorb oxygen simultaneously generating carbon dioxide to maintain the carbon dioxide content at 10% in the culture atmosphere during 24 - 48 hours after sealing. In the practical use, additional deoxidizers or carbon dioxide-generators can be used in combination of said compositions according to the conditions of temperature, moisture etc. In such a case every components have to be decreased in the amounts according to additional components, provided that removed oxygen is twice the volume of the generated carbon dioxide.

So far as this principle is held any kinds of variation are allowed. So, the composition may be composed of a combination of two groups of agents as follows: One of them being a group of the deoxidizing agent incapable of generating carbon dioxide, the other being a group with both capable of generating carbon dioxide and removing oxygen; provided that, e.g., the volume of generated carbon dioxide of the latter group be equivalent to the oxygen removed (or absorbed) and, at the same time, the speed of removing oxygen with the former group be approximately the same as that of the latter. Analogously, various combinations of the components are possible in correspondance to the difference of the reaction speed in case of the components having various reaction rates, i.e., different ratios between deoxidizing speed and carbon dioxide-generating speed.

As the deoxidizers some iron compounds are also well known. Japanese patent Kokoku publication Nos. 54(1979)-438, 54(1979)-439, 54(1979)-471, 54(1979)-472, 54(1979)-476 etc disclose various ferrous deoxidizers. Those are applicable in this invention. The partinent disclosures as disclosed in the above JP Kokoku publications are herewith incorporated in this Specification. A combination of ascorbic acid or its salts / alkali hydroxide/ accelerators is also a useful deoxidizer. This composition includes alkali hydroxides (Calcium hydroxide is preferable) instead of alkali carbonates and alkali hydrogen carbonates in the compositions (hereinbefore mentioned) of the carbon dioxide-generating deoxidizers. In this case, as a composition of carbon dioxide-generating deoxidizer, it is possible to employ such a composition that generates carbon dioxide equivalent to the volume of removed oxygen. To this end, e.g., following composition
is useful.

|  | parts by weight |
|---|---|
| soudium-L-ascorbate | 6 |
| $NaHCO_3$ | 6 |
| $Na_2CO_3 \cdot 10H_2O$ | 6 |
| $FeSO_4 \cdot 7H_2O$ | 2 |
| active carbon | 6 |

There are two types of the carbon dioxide generators.
One is a carbonate such as sodium hydrogen carbonate, sodium
carbonate, potassium carbonate, sodium sesquicarbonate and the
like. The other is acidic compounds capable of generating
carbon dioxide on dissolving in water resulting in a weak acidic
pH (pH<6) such as ascorbic acid, tartaric acid, succinic acid,
malic acid, fumaric acid, lactic acid etc, preferably ascorbic
acid.

When the net-volume of the culture vessels is known, a
further variation of the process is possible. Namely, it is
possible to apply a combination of a deoxidizer removing oxygen
up to 10 % of the net volume of the culture vessel with a carbon
dioxide-generating deoxidizer generating the same volume of
carbon dioxide as the oxygen removed.

A significant merit of the agents of the invention resides
in that it is unnecessary to weigh out many sorts of components
with different ratios according to each culture vessel. All that
the users have to do is to enclose the composition of an amount
not less than the minimum amount for removing all oxygen of the
vessels in the vessels. Therefore, the inventive process is
significantly convenient on use.

It makes more convenient to sealingly package the agents in a bag with double layers. The bag consists of an outer bag made of gas-impermeable plastic film and an inner bag made of gas-permeable sheet, e.g., plastic film, paper or cloth. For the purpose of long storage, additional covering with an aluminium foil or a laminated plastics-metal foil is also useful.

On use, the outer bag is cut open, then the inner bag is enclosed in a calture vessel which may be a large gas-impermeable bag or envelope.

Another merit of the invention resides in that the process requires no special vessel having pressure resistance such as vacuum vessel as is conventional in the prior art. The cultivation proceeds in a gas-impermeable envelope under the condition of a slightly reduced pressure of 10 % $CO_2$ in place of 21 % $O_2$. The gas-impermeable envelope may be conventional one. The same envelope for storing the agents before use may be used as the culture vessel. Using the agents of the invention, when all the oxygen amounted to 21 % of the air is removed, carbon dioxide of its half volume is consequently generated. Thus the content of carbon dioxide theoretically results in 11.7 % at the final stage. In this process the carbon dioxide content practically provides a range of 10 ± 2 %. The anaerobic cultivation can be performed at the content of 10 % carbon dioxide promptly as occasion demands and in each culture vessel of a desired volume as well as indivisually. Through a transparent envelope employed as the culture vessel, every specimen can be observed without opening the envelope sealed for culturing.

The test results according to the inventive process are shown as follows:

[Test 1] A prescribed amount of the ascorbate composition of carbon dioxide-generating deoxidizers hereinbefore mentioned as (A) was enclosed in a sealed culture vessel of 250ml. Both contents of oxygen and carbon dioxide were measured and found 10.2% carbon dioxide with oxygen of less than 0.1% after 24 hours, and 10.4% carbon dioxide with oxygen of less than 0.1% after 48 hours.

[Test 2] Each one package of the deoxidizers sold by Mitsubishi Gas Chemical Co., Ltd. with the commercial name of "Ageless S-50" and "Ageless S-30", and 2 packages of the carbon dioxide-generating deoxidizer sold by Toppan Printing Co., Ltd. with the commercial name of "C-500" were enclosed in the culture vessel of a net volume of 250ml. After 3 hours at 37°C, 9.8% carbon dioxide and less than 0.1% oxygen were confirmed. From 24 hours to 48 hours after enclosure, an anaerobic atmosphere (less than 0.1% oxygen) was maintained and the carbon dioxide content varied from 10.1% to 9.2%.

[Test 3] In an analogous manner with Test 2, various volumes of culture vessels raging from 200 to 500ml were tested exhibiting similar results. Judging from these test results, this process quickly accomplishes the anaerobic condition and maintains approximately 10% $CO_2$ content during a period of time sufficient for the anaerobic culture regardless of the vessel volume when sufficient amount of the agent is applied.

A further simple embodiment will be hereinbelow disclosed with reference to a drawing. An outer envelope 1 is made of a transparent and gas-impermeable film with the oxygen-permeability of less than $50cc/m^2 \cdot atm \cdot dry$ and preferably less than $20cc/m^2 \cdot atm \cdot dry$. The film encompasses, e.g., nylon, polypropylene- or polyester-film each coated with polyvinylidene chloride, polyvinylidene film, copoliner film of vinyl alcohol with ethylene and the like. One end 1b of the envelop is being sealed. A petri dish 6 after innoculation of a specimen on a culture medium 7 is enclosed in the outer envelope 1. Then the permeable inner bags 2, 3 containing the compositions of carbon dioxide-generating deoxidizers 4, 5 are also enclosed in the envelope together with a known indicator 9 for the detection of anaerobic condition in color. Then, the open side 1a is sealed with a clip 8. One or two, or any more pieces of the inner bags 2, 3 (2 pieces being shown in the drawing) may be used at need. Other known manners for sealing or gas-tightly closing like heat sealing etc. are applicable for sealing the open end of the envelope.

About 10% of carbon dioxide is generated at the anaerobic stage, hence the envelope 1 is not intensely crushed and the conventional petri dishes can resist well against the pressure. Thus special pressure vessels are unnecessitated. Every petri dishes can be observed from outside and the examiner can individually check the growth of colony from one dish to another dish at any time through the transparent envelope without opening it. Control of the temperature is easy because of small size and the thin layer of the envelope made of the gas-impermeable film contrary to that of the conventional pressure vessel or vaccum vessel.

Besides it, as the reactions in this process are neither endothermic nor exothermic, the temperature remains steady without special care as well as easily controlable at need. Usually, the moisture is not aspired, nor expired by the reaction according to the preferred embodiment.

[Example 1]

As a carbon dioxide-generating deoxidizer the composition A-1 hereinabove mentioned was used to maintain the atmosphere of $10 \pm 2$ % carbon dioxide and less than 0.1% oxygen during 6 - 48 hours after inoculation on the GAM agar culture medium. Following strains were cultured for 48 hours at 37°C, and the growth of these strains were observed and compared with respect to the size of the colony:

Bacteroides, Fusobacterium, Clostridium, Propionibacterium, Lactobacillus, Peptococcus, Peptostreptococcus and Veillonella. All of them grew up each to form a sufficiently large colony and detected. In the same manner another 30 kinds of anaerobic strain were tested and detected successfully.

[Comparative Test 1]

Cultivation was carried out in an atmosphere of 5% carbon dioxide otherwise in the similar manner to Example 1. Fusobacterium could not be detected.

[Comparative Test 2]

Cultivation was performed at a content of 20% carbon dioxide with less than 0.1% oxygen. Most of strains could not be detected because of their poor growth.

Accordingly, those tests as disclosed show the atmosphere containing 10% carbon dioxide can effectively work for detecting more strains which are contained in the specimens clinically sampled. Thus the process of the present invention has turned out useful particularly in the field of clinical culture testing.

- 14 -

0120111

CLAIMS

What is claimed is:

1. A process for culturing anaerobic bacteria wherein an agent enclosed in a gas-permeable inner bag is put in an airtightly closed culture vessel, the agent comprising a composition which is capable of removing oxygen and generating carbon dioxide in a volumetric ratio of the removed oxygen approximately twice as much as the generated carbon dioxide.

2. A process as defined in Claim 1, wherein said agent is put in an amount sufficient to bring the interior atmosphere in the culture vessel to a substantially oxygen-free state, the resultant state being maintained at the carbon dioxide concentration of 10 $\pm$ 2 % by volume.

3. A process as defined in Claim 1 or 2, wherein said closed culture vessel is an oxygen-impermeable and transparent bag with its open end being gas-tightly closed or sealed.

4. An agent for preparing an anaerobic culture atmosphere for culturing anaerobic bacteria comprising a composition which is capable of removing oxygen and generating carbon dioxide in a volumetric ratio of the removed oxygen approximately twice as much as the generated carbon dioxide.

5. An agent as defined in Claim 4, wherein the composition is of the type mainly comprising:

(a) ascorbic acid, ascorbic salt or a mixture thereof,

(b) carbonate or hydrogen carbonate of alkali , or a mixture thereof, and

(c) reaction accelerator.

6. An agent as defined in Claim 4, wherein said composition is of the type mainly comprising:

(a) powdery iron,

(b) ferrous sulfate, and

(c) carbonate or hydrogen carbonate of alkali, or a mixture thereof.

7. An agent as defined in Claim 4, wherein said composition substantially consisting of a deoxidizer and a carbon dioxide-generating deoxidizer having approximately the same deoxidizing speed with said deoxidizer, the carbon dioxide-generating deoxidizer being capable of removing oxygen at the same volumetric ratio as the generated carbon dioxide.

8. An agent as defined in Claim 7, wherein said deoxidizer and said carbon dioxide-generating deoxidizer are separately reserved in each oxygen-impermeable outer bag before use.

# FIG. 1

European Patent
Office

EUROPEAN SEARCH REPORT

0120111

Application number

EP 83 10 3126

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | GB-A-2 083 496 (MITSUBISHI GAS CHEMICAL CIE. INC.) <br> * Claims 1,2,4,8,14,15; page 2, lines 28-32; table I; examples * <br><br> --- | 1-8 | C 12 M 1/04 |
| A | CHEMICAL ABSTRACTS, vol. 92, no. 1, January 1980, page 460, no. 4728r, Columbus, Ohio, USA <br> & JP - A - 79 105 288 (TOPPAN PRINTING CO., LTD.) 18-08-1979 * <br><br> ----- | | |

TECHNICAL FIELDS
SEARCHED (Int. Cl. ³)

C 12 M
B 01 J
C 12 N

The present search report has been drawn up for all claims

| Place of search THE HAGUE | Date of completion of the search 16-09-1983 | Examiner COUCKE A.O.M. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82